# EUROPEAN PATENT APPLICATION

(11) **EP 4 538 259 A1**
(43) Date of publication of application: **16.04.2025**
(21) Application number: 23383031.4
(22) Date of filing: 09.10.2023
(51) Int. Cl.: C07D 249/08, C07D 403/12

(54) **PROCESSES FOR THE PREPARATION OF DEUCRAVACITINIB**

(71) Applicant: Farmhispania Group, S.L., 50016 Zaragoza (ES)
(72) Inventor: ETAYO PÉREZ, Pablo, 50010 ZARAGOZA (ES); PUJOL OLLÉ, Xavier, 08025 BARCELONA (ES); MOLINA NIETO, Juan, 08922 SANTA COLOMA (ES)
(74) Representative: ZBM Patents - Zea, Barlocci & Markvardsen

(57) **Abstract**

The invention relates to processes for the preparation of deucravacitinib of formula (XI), or a pharmaceutically acceptable salt thereof. It also relates to intermediates of formula (V') and (V") as defined herein useful in the synthesis of this molecule.

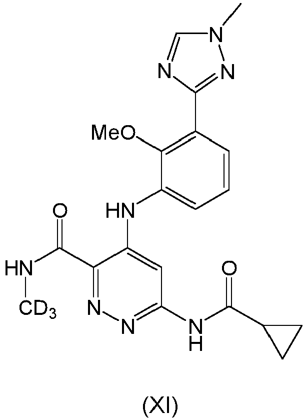

## Description

### Technical Field

The invention relates to processes for the preparation of deucravacitinib and of intermediates useful in the synthesis of this molecule.

### Background Art

Deucravacitinib is a Tyk2 inhibitor which was approved in United States in 2022 and in Europe in 2023 for the treatment of moderate to severe plaque psoriasis in adults who are candidates for systemic therapy. Deucravacitinib's chemical name is 6-(cyclopropane-carboxamido)-4-((2-methoxy-3-(1-methyl-1H-1,2,4-triazol-3-yl)phenyl)amino)-N-(trideuteromethyl)pyridazine-3-carboxamide and it was first disclosed in WO2014/0074661.

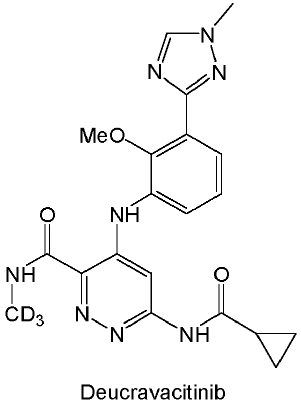

Psoriasis is a chronic, non-communicable, painful, immunologically-mediated, disfiguring and disabling inflammatory skin disease with great negative impact on patients' quality of life (QoL). It is characterized by marked inflammation and thickening of the epidermis that result in thick, scaly plaques involving the skin. Psoriasis may be classified according to morphologic and clinical presentation: plaque psoriasis, guttate psoriasis, erythrodermic psoriasis, generalized pustular and localized pustular psoriasis, and inverse or intertriginous psoriasis.

Plaque psoriasis is the most common form of the disease. Areas of the body that are frequently involved include the scalp, elbows, knees, buttocks, and genitalia. The extent of skin involved varies among affected individuals and is a primary determinant of severity.

In patients with plaque psoriasis, approximately 80% have mild to moderate disease, with 20% having moderate to severe disease. Nails of hands and feet are often involved. Nail psoriasis presents a spectrum of challenges to patients: pain associated with nail bed hyperkeratosis, functional deficits and cosmetic disfigurement.

Several synthetic processes for preparing deucravacitinib and intermediates thereof have been disclosed. Preparation of deucravacitinib was first disclosed in WO2014/0074661. The process disclosed therein requires several synthetic steps and it is characterized in that the introduction of the methyl-*d*₃-amine moiety is carried out before the coupling with the intermediate (VI):

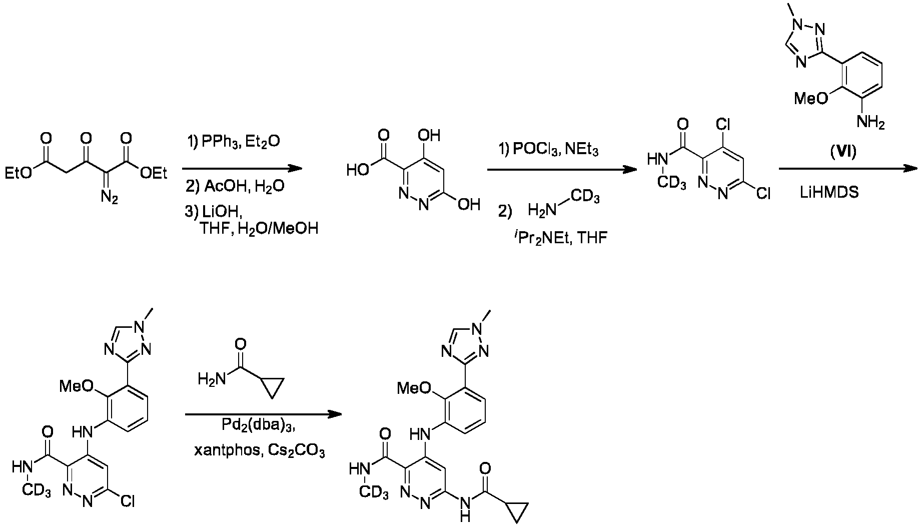

This process has some drawbacks; it requires the hydrolysis of the ester group (ethyl ester) with LiOH and subsequent acidification of the generated lithium carboxylate, which implies an additional step in the process. Moreover, introducing the deuterated amine at an early stage is not cost-effective.

WO2018/0183649 refers to a process wherein introduction of the methyl-*d*₃-amine moiety is carried out in presence of activating and coupling agents:

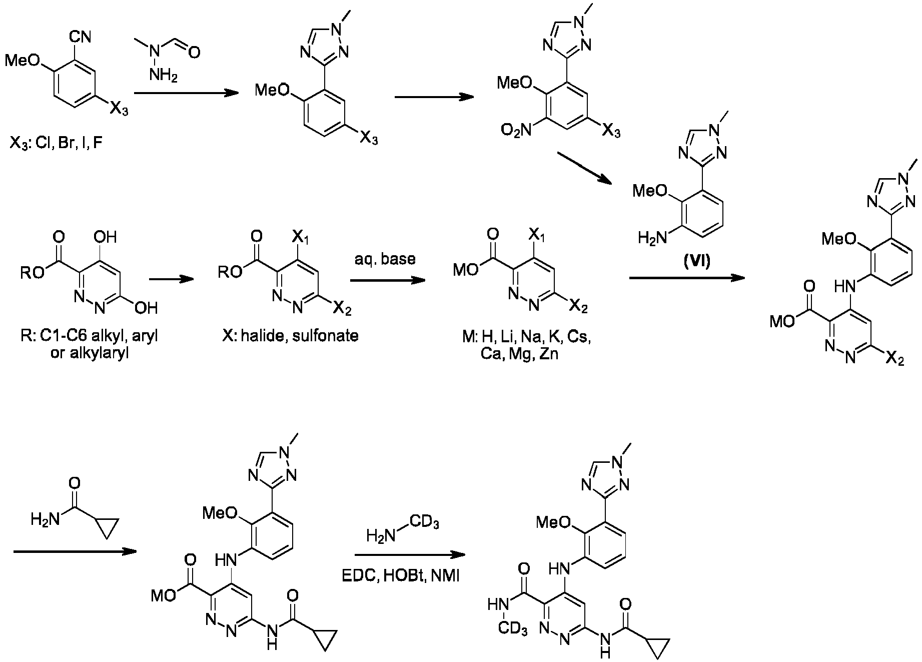

This process implies an additional step of hydrolysis/saponification of the ester group. Furthermore, the use of activating and coupling agents (EDC·HCI and HOBt) generates the corresponding reagent byproducts which are typically difficult to be fully removed from the amidation product.

Further, WO2023/102085 discloses a process for the preparation of deucravacitinib comprising reacting the ethyl ester intermediate (XIII) with deuterated methylamine to give intermediate (XVII), and subsequently reacting the latter with cyclopropane carboxamide to afford the final product:

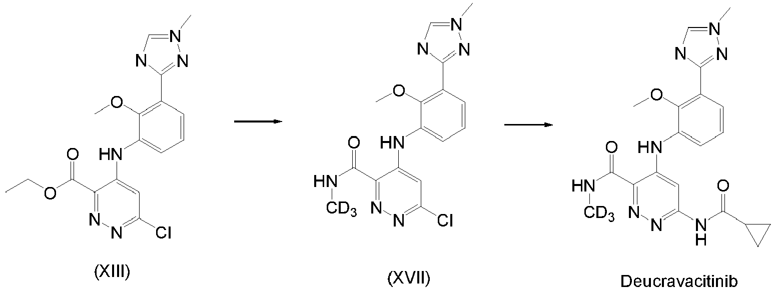

Example 31 of the PCT application illustrates the preparation of intermediate (XVII) in the presence of lithium bis(trimethylsilyl)amide (LiHMDS) and using tetrahydrofuran as a solvent. Compound (XVII) is said to be recovered in 75% yield. However, in the hands of the present inventors, when trying to reproduce this example, it was found that the yield of this reaction was indeed much lower, around 34%. This low yield is a major disadvantage when the process is to be applied on an industrial scale taking into account that intermediate (XVII) is a very advanced intermediate in the deucravacitinib route of synthesis, and therefore a more valuable one compared to other intermediates used at early stages of the synthesis. Furthermore, in the hands of the present inventors the purity of the obtained compound was around 88%, which ultimately has also an impact on the overall performance of the process. Therefore, due to the poor results of the transformation of intermediate (XIII) into intermediate (XVII), the process described WO2023/102085 is not of practical application on an industrial scale.

Therefore, there is still the need to develop an alternative process for obtaining deucravacitinib which overcomes the problems associated with the known processes belonging to the state of the art, and which can be conveniently implemented on an industrial scale.

### Summary of Invention

The inventors have developed a new and safer process for the preparation of deucravacitinib which involves fewer synthetic steps than the processes cited above. Besides, the developed process avoids the use of activating and coupling agents, which give rise to by-products that are difficult to remove. The process proceeds with good isolated yields and high chemical purities. These features make the process of the invention especially suitable to be industrially implemented.

In particular, the inventors have found that when the methyl ester intermediate (IX) (instead of the ethyl ester intermediate) was reacted with deuterated methylamine under very specific conditions, the yield and purity of the obtained amide intermediate were notably improved in comparison to what is disclosed in WO2023/102085. Hence, as illustrated in the examples, the reaction of the methyl ester intermediate (IX) with deuterated methylamine under very mild conditions, i.e., in the presence of cesium carbonate and using dimethylformamide (DMF) as a solvent, gave the amide intermediate in very good yield (84%) and purity (>99% a/a) at pilot scale (270 g). This was completely unexpected by the inventors taking into account that the analog reaction with the ethyl ester intermediate at laboratory scale (0.5 g) and under much stronger conditions (LiHMDS in THF), the amide intermediate was only obtained in 30% yield and purity below 90%. In fact, LiHMDS is a highly sensitive reagent to oxygen and humidity and requires working in anhydrous conditions and strict inert atmosphere.

Further, when the reaction was carried out with the ethyl intermediate as disclosed in WO2023/102085, the analysis of the separated aqueous layer confirmed the presence of the free carboxylic acid derivative formed through undesired ester hydrolysis as a major reaction side product. This results not only in low yield and purity, but in the formation of carry-over impurities in the next step of the process, resulting in a final product having lower purity.

Thus, a first aspect of the invention relates to a process for the preparation of deucravacitinib of formula (XI) or a pharmaceutically acceptable salt thereof, 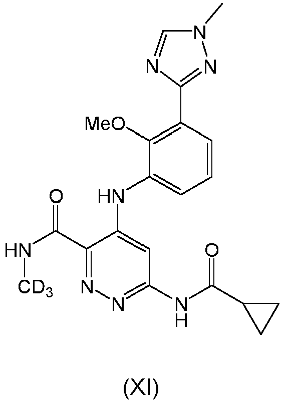 which comprises:
a) reacting a compound of formula (IX), or a salt thereof, 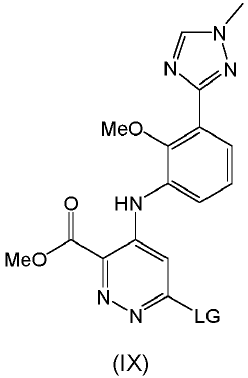 with methyl-*d*₃-amine, or a salt thereof, in the presence of cesium carbonate in dimethylformamide to give a compound of formula (X) or a salt thereof; 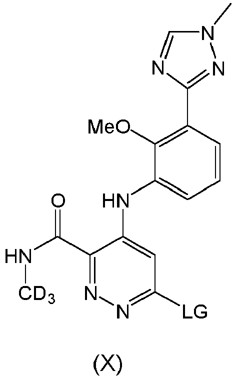 wherein LG is a leaving group selected from the group consisting of halogen, -OSO₂R₁, and -OCOR₁, and R₁ is selected from the group consisting of -(C₁-C₄)alkyl, -(C₁-C₄)haloalkyl, -(C₁-C₄)alkyl(C₆-C₁₂)aryl, -(C₁-C₄)alkyl(C₄-C₁₂)heteroaryl, -(C₆-C₁₂)aryl, and -(C₄-C₁₂)heteroaryl; wherein the (C₆-C₁₂)aryl and (C₄-C₁₂)heteroaryl moieties are optionally substituted with one or more (C₁-C₄)alkyl or (C₁-C₄)haloalkyl groups;
b) reacting the compound of formula (X), or a salt thereof, with cyclopropanecarboxamide, or a salt thereof, to give a compound of formula (XI); and
c) optionally converting deucravacitinib of formula (XI) into a pharmaceutically acceptable salt thereof.

A second aspect of the invention relates to a process for the preparation of a compound of formula (V'), or a salt thereof, 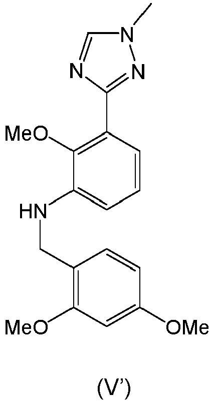 which comprises:
iv) reacting the compound of formula (IV), or a salt thereof, with 2,4-dimethoxybenzylamine, or a salt thereof, in presence of metallic catalyst.

A third aspect of the invention relates to a process for the preparation of a compound of formula (V"), or a salt thereof, 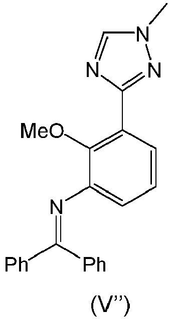 which comprises:
iv') reacting the compound of formula (IV), or a salt thereof, with benzophenone imine, in presence of metallic catalyst.

The present invention also relates to new intermediates of formula (V'), and formula (V"), which can be efficiently used in the process for the preparation of deucravacitinib.

Accordingly, a third aspect of the invention relates to a compound of formula (V'), and a fourth aspect of the invention relates to a compound of formula (V").

Processes for the preparation of a compound of formula (V'), and a compound of formula (V") also form part of the invention.

### Brief Description of Drawings

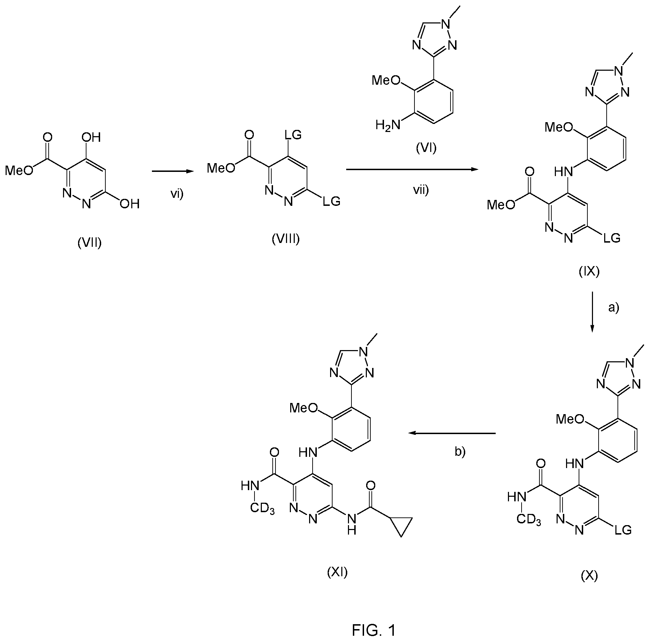

FIG. 1 shows an embodiment of the invention for the preparation of deucravacitinib of formula (XI).

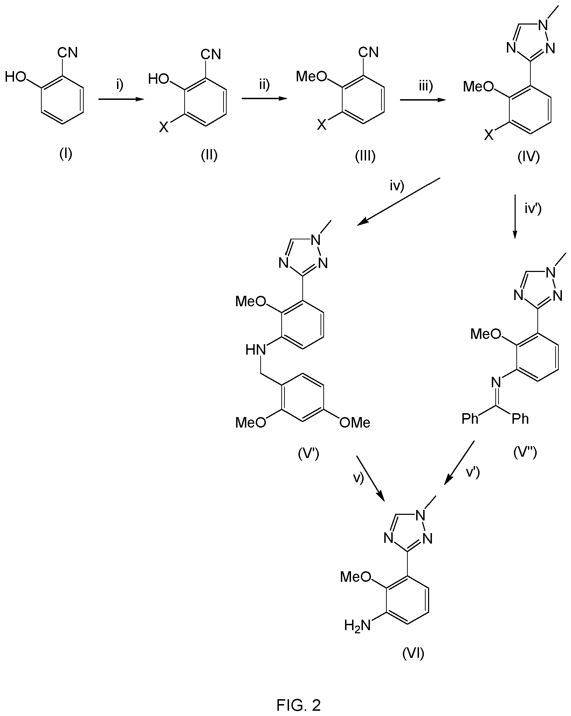

FIG. 2 shows an embodiment of the invention for the preparation of an intermediate of formula (VI).

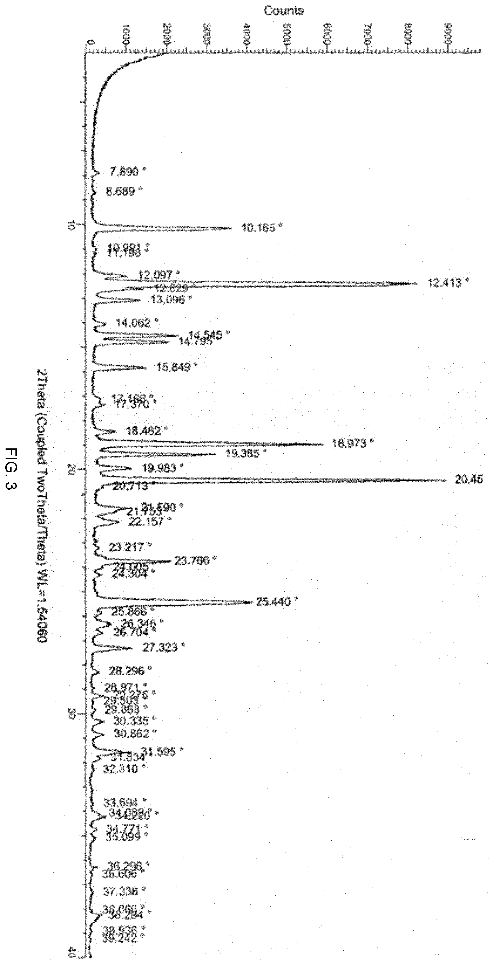

FIG. 3 shows the XRPD powder diffractogram of deucravacitinib Form A.

### Detailed description of the invention

All terms as used herein in this application, unless otherwise stated, shall be understood in their ordinary meaning as known in the art. Other more specific definitions for certain 30 terms as used in the present application are as set forth below and are intended to apply throughout the description and claims.

The term "about" or "around" as used herein refers to a range of values ± 10% of a specified value. For example, the expression "about 10" or "around 10" includes ± 10% of 10, i.e., from 9 to 11.

For the purposes of the invention, the expression "room temperature" means a temperature from 20 to 25 °C.

The term halogen or halide means fluoro, chloro, bromo or iodo.

The terms "trideuteromethylamine", "methyl-*d*₃-amine" and "deuterated methylamine" are used herein interchangeably and refer to the compound CD₃NH₂. For the purposes of the invention deuterated methylamine or a salt thereof such as e.g., the hydrochloride may be used.

The term "(C₁-C₄)alkyl" refers to a linear or branched saturated hydrocarbon group having from 1 to 4 carbon atoms. By way of example, mention may be made of methyl, ethyl, propyl, isopropyl, butyl, isobutyl, sec-butyl, or tert-butyl groups.

The term "(C₁-C₄)haloalkyl" refers to a (C₁-C₄)alkyl group as previously defined in which one or more hydrogen atoms are replaced by same or different halogen atoms. By way of example, mention may be made of -CF₃, -CH₂CF₃, -CH₂Cl, or -CH₂CCl₃.

The term "(C₆-C₁₂)aryl" refers to an aromatic carbocyclic mono- or bicyclic ring system comprising from 6 to 12 carbon ring atoms. Non-limiting examples of aryl moieties include phenyl and naphthyl.

The term "(C₄-C₁₂)heteroaryl" refers to a heteroaromatic carbocyclic mono- or bicyclic ring system comprising from 4 to 12 carbon ring atoms, wherein one or more of the ring members, particularly 1, 2, 3, or 4 ring members, are selected from NH, N, O, and S, where chemically possible. The remaining ring members of the heteroaromatic ring are independently selected from C, CH, O, N, NH, and S.

The term "(C₁-C₄)alkyl(C₆-C₁₂)aryl" refers to a (C₁-C₄)alkyl group as defined herein which is attached to the parent moiety and which is further substituted with a (C₆-C₁₂)aryl group as defined herein. Non-limiting examples of (C₁-C₄)alkyl(C₆-C₁₂)aryl moieties include benzyl and methylnaphthyl.

The term "(C₁-C₄)alkyl(C₄-C₁₂)heteroaryl" refers to a (C₁-C₄)alkyl group as defined herein which is attached to the parent moiety and which is further substituted with a (C₄-C₁₂)hereroaryl group as defined herein.

The term "Lewis acid" as used herein refers to a compound or ionic species which can accept an electron pair from a donor compound. By way of example, mention may be made of MgCl₂, MgSO₄, MgBr₂, etc.

The term "-OTf" refers to triflate or trifluoromethanesulfonate, which means -OSO₂CF₃, which is the anion of trifluoromethanesulfonic acid. The term "-OTs" refers to tosylate, which means the anion -OSO₂C₇H₇, which is the anion of p-toluenesulfonic acid. The term "-OMs" refers to mesylate which means the anion -OSO₂CH₃, which is the anion of methanesulfonic acid.

The term "halogenating agent" refers to a substance that can transfer one halogen atom to the compound with which they are reacting.

The term "telescoped process" as used herein refers to a sequential one-pot synthesis whereby the reagents are added to the reactor one at a time and without work-up.

As mentioned above the first aspect of the invention relates to a process for the preparation of deucravacitinib.

Deucravacitinb of formula (XI) may be converted into a pharmaceutically acceptable salt thereof. The term "pharmaceutically acceptable salts" encompasses any salt formed from pharmaceutically acceptable non-toxic acids including inorganic or organic acids such as for example acetic, trifluoroacetic, benzenesulfonic, benzoic, camphorsulfonic, citric, ethansulfonic, oxalic, fumaric, gluconic, glutamic, hydrobromic, hydrochloric, orthophosphoric, lactic, maleic, malic, mandelic, methanesulfonic, phosphoric, succinic, sulfuric, tartaric, p-toluenesulfonic, glutamic, aspartic acid, and the like. There is no limitation regarding the salts, except that if used for therapeutic purposes, they must be pharmaceutically acceptable. Furthermore, deucravacitinib salts may be used to purify deucravacitinib.

The preparation of pharmaceutically acceptable salts of deucravacitinb can be carried out by methods known in the art. For instance, they can be prepared by reacting deucravacitinb with a stoichiometric amount of the appropriate pharmaceutically acceptable acid in water, in an organic solvent or in a mixture of them. The compounds of formula (XI) and their salts may differ in some physical properties, but they are equivalent for the purposes of the present invention.

Deucravacitinib and/or salts may be in crystalline form either as free solvation compound or as solvate (*e.g*., hydrate). All these forms are within the scope of the present invention. Methods of solvation are generally known within the art. In general, the solvated forms with pharmaceutically acceptable solvents such as water, ethanol and the like are equivalent to the unsolvated form for the purposes of the invention.

The obtained deucravacitinib may be crystallized from a suitable solvent selected from the group consisting of acetonitrile (ACN), methyl ethyl ketone (MEK), methanol (MeOH), toluene, ethyl acetate (EtOAc), dichloromethane (DCM), ethanol (EtOH), tetrahydrofuran (THF), 2-methyltetrahydrofuran, isopropanol, dimethyl sulfoxide (DMSO), N-methyl-2-pyrrolidone, water and mixtures thereof, more particularly ethanol/water. Such a crystallization procedure provides a well-defined polymorphic form of deucravacitinib in a reproducible manner, which is consistent with deucravacitinib crystalline Form A which is further defined in the patent application WO2018/0183656.

In all embodiments of the invention referring to the compound of formula (XI), the pharmaceutically acceptable salts thereof and the polymorphic forms, either of any of the compounds of formula (XI) or of any of their pharmaceutically acceptable salts are always contemplated even if they are not specifically mentioned.

As mentioned above the first aspect of the invention relates to a process for the preparation of deucravacitinib of formula (XI) or a pharmaceutically acceptable salt thereof, which comprises:
a) reacting a compound of formula (IX), or a salt thereof, with methyl-*d*₃-amine, or a salt thereof, in the presence of cesium carbonate in dimethylformamide to give a compound of formula (X) as defined herein;
b) reacting the compound of formula (X), or a salt thereof, with cyclopropanecarboxamide, or a salt thereof to give a compound of formula (XI); and
c) optionally converting deucravacitinib of formula (XI) into a pharmaceutically acceptable salt thereof.

Step a) comprises the reaction of methyl-*d*₃-amine with a compound of formula (IX). In one embodiment, optionally in combination with one or more features of the various embodiments described above or below, methyl-*d*₃-amine, or a salt thereof, is used in an amount from 1 to 15 molar equivalents, particularly from 1 to 5 molar equivalents, more particularly from 1 to 3 equivalents, and even more particularly about 2.5 molar equivalents, relative to the compound of formula (IX).

The amount of the base (cesium carbonate) used in step a) may be generally from 1 to 5 molar equivalents, more particularly from 1 to 3 equivalents, and even more particularly about 2.4 molar equivalents, relative to compound of formula (IX)..

In another embodiment, optionally in combination with one or more features of the various embodiments described above or below, in step a) methyl-*d*₃-amine and cesium carbonate are used each in an amount from 1 to 5 molar equivalents, relative to the compound of formula (IX).

Step a) can be carried out at a suitable temperature, *e.g.,* at room temperature, and with a reaction time generally from 2 to 24 h.

According to one embodiment, optionally in combination with one or more features of the various embodiments described above or below, in the compound of formula (IX), LG is selected from the group consisting of a halogen, -OTf, -OTs and -OMs.

In another embodiment, optionally in combination with one or more features of the various embodiments described above or below, in the compound of formula (IX), LG is a halogen, more particularly LG is chloro, i.e., the compound of formula (IX) is a compound of formula (IXa):

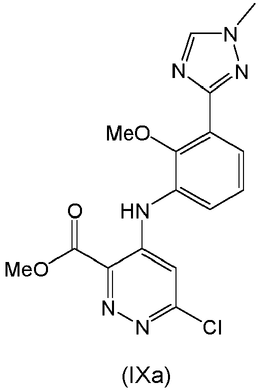

In another embodiment, optionally in combination with one or more features of the various embodiments described above or below, in the compound of formula (X), LG is a halogen, more particularly LG is chloro, *i.e.,* the compound of formula (IX) is a compound of formula (Xa):

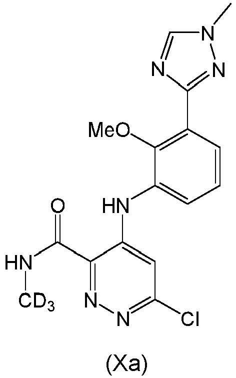

Step b) comprises the reaction of cyclopropanecarboxamide with a compound of formula (X). In one embodiment, optionally in combination with one or more features of the various embodiments described above or below, cyclopropanecarboxamide, or a salt thereof, is used in an amount from 1 to 3 molar equivalents, particularly about 1.5 molar equivalents, relative to the compound of formula (X).

Step b) can be carried out in a suitable solvent such as organic solvent which is not particularly limited, and at a suitable temperature, e.g., at room temperature, and with a reaction time generally from 3 to 18 h. In one embodiment, optionally in combination with one or more features of the various embodiments described above or below, step b) is carried out in an organic solvent selected from the group consisting of *N,N-*dimethylformamide, 1,4-dioxane, *tert*-butanol, dichloromethane, dichloroethane, toluene, acetonitrile, tetrahydrofuran, 2-methyltetrahydrofuran, ethyl acetate, and combinations thereof, more particularly, the solvent is 1,4-dioxane.

Step b) may be performed in the presence of a base which is not particularly limited. The amount of the base may be generally from 1 to 3 molar equivalents, particularly about 2.5 molar equivalents, relative to compound of formula (X). In one embodiment, optionally in combination with one or more features of the various embodiments described above or below, the base used in step b) is selected from the group consisting of sodium carbonate, sodium hydrogen carbonate, potassium carbonate, potassium hydrogen carbonate, potassium phosphate, cesium carbonate, 1,8-diazabicyclo[5.4.0]undec-7-ene (DBU), and combinations thereof, more particularly, the base is potassium phosphate.

Step b) may be performed in the presence of a palladium catalyst in an amount generally from 0.005 to 0.020 molar equivalents, particularly about 0.075 molar equivalents, relative to compound of formula (X), and a phosphane ligand in an amount generally from 0.010 to 0.050 molar equivalents, particularly about 0.15 molar equivalents, relative to compound of formula (X). In one embodiment, optionally in combination with one or more features of the various embodiments described above or below, step b) is carried out in the presence of a catalyst and a phosphane ligand, more particularly, the catalyst is selected from the group consisting of tris(dibenzylideneacetone)dipalladium(0) (Pd₂(dba)₃), Pd(OAc)₂, Pd(PPh₃)₂Cl₂, Pd(PPh₃)₄, Pd(dba)₂ and Pd(dppf)Cl₂, and the phosphane ligand selected from the group consisting of 1,1'-ferrocenediyl-bis(diphenylphosphine) (dppf), (±)-2,2'-bis(diphenylphosphino)-1,1'-binaphthalene (BINAP), (2R)-1-[(1R)-1-[bis(1,1-dimethylethyl)phosphino]ethyl]-2-(dicyclohexylphosphino)ferrocene (Josiphos), 2-dicyclohexylphosphano-2'-(*N,N-*dimethylamino)biphenyl (DavePhos), and 4.5-bis(diphenylphosphano)-9,9-dimethylxanthene) (Xantphos), and even more particularly the catalyst is 1,1'-ferrocenediyl-bis(diphenylphosphine) (dppf), Pd(OAc)₂ and the phosphane ligand is 1,1'-ferrocenediyl-bis(diphenylphosphine) (dppf).

According to one embodiment, optionally in combination with one or more features of the various embodiments described above or below, in the process of the first aspect previously to step a), the process comprises:
vii) reacting the compound of formula (VIII), or a salt thereof, 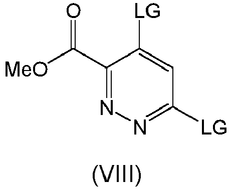 wherein each LG is independently a leaving group selected from the group consisting of halogen, -OSO₂R₁, and -OCOR₁, and R₁ is selected from the group consisting of -(C₁-C₄)alkyl, -(C₁-C₄)haloalkyl, -(C₁-C₄)alkyl(C₆-C₁₂)aryl, -(C₁-C₄)alkyl(C₄-C₁₂)heteroaryl, - (C₆-C₁₂)aryl, and -(C₄-C₁₂)heteroaryl; wherein the (C₆-C₁₂)aryl and (C₄-C₁₂)heteroaryl moieties are optionally substituted with one or more (C₁-C₄)alkyl or (C₁-C₄)haloalkyl groups;
with a compound of formula (VI), or a salt thereof, 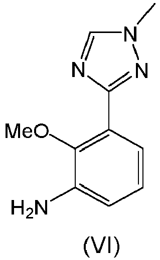 to obtain a compound of formula (IX), or a salt thereof.

Step vii) comprises the reaction of a compound of formula (VIII) with a compound of formula (VI) or a salt thereof. In one embodiment, optionally in combination with one or more features of the various embodiments described above or below, the compound of a formula (VIII), or a salt thereof, is used in an amount from 1 to 3 molar equivalents, more particularly about 1.3 molar equivalents, relative to the compound of formula (VI).

Step vii) can be carried out in a suitable solvent such as organic solvent which is not particularly limited, and at a suitable temperature, e.g., by working at temperature from 25 to 35 °C, and with a reaction time generally from 3 to 18 h. In one embodiment, optionally in combination with one or more features of the various embodiments described above or below, step vii) is carried out in an organic solvent selected from the group consisting of *N,N*-dimethylformamide, *tert*-butanol, 1,4-dioxane, dichloromethane, dichloroethane, toluene, acetonitrile, tetrahydrofuran, 2-methyltetrahydrofuran, ethyl acetate, and combinations thereof, more particularly, the solvent is *tert*-butanol.

Step vii) may be performed in the presence of a base which is not particularly limited. The amount of the base may be generally from 1 to 5 molar equivalents, particularly about 1.5 molar equivalents, relative to compound of formula (VI). In one embodiment, optionally in combination with one or more features of the various embodiments described above or below, the base used in step vii) is selected from the group consisting of triethylamine, *N,N*-diisopropylethylamine (DIPEA), pyridine, N-methylmorpholine (NMM), *N-*methylimidazole (NMI), and combinations thereof, even more particularly the base is *N,N-*diisopropylethylamine (DIPEA).

Step vii) may be carried out in the presence of one or two Lewis acids which are not particularly limited. The amount of each Lewis acid may be generally from 1 to 3 molar equivalents, particularly about 1.1 or 2.0 molar equivalents, relative to compound of formula (VI). In one embodiment, optionally in combination with one or more features of the various embodiments described above or below, the Lewis acids of step vii) are selected from the group consisting of AgOTf, AgNO₃, Ag₂SO₄, AgOAc, MgCOs, MgCl₂, MgSO₄, and MgBr₂, and more particularly are AgOTf and MgSO₄.

According to one embodiment, optionally in combination with one or more features of the various embodiments described above or below, in the process of the first aspect previously to step vii), the process comprises:
vi) converting a compound of formula (VII), or a salt thereof, 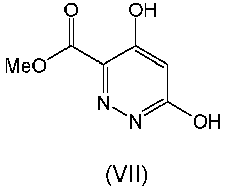 into compound of formula (VIII), or a salt thereof as defined herein.

According to one embodiment, optionally in combination with one or more features of the various embodiments described above or below, in the compound of formula (VIII), each LG is independently an halogen, more particularly both LG are the same, and even more particularly each LG is chloro, i.e., the compound of formula (VIII) is a compound of formula (Villa):

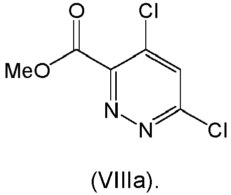

Step vi) can be carried out in the presence of a suitable halogenating agent (when LG is halogen), a sulfonylating agent (when LG is a sulfonate) or an acylating agent (when LG is an ester). The halogenating agents, sulfonylating agents, and acylating agents that can be used are not particularly limited.

In one embodiment, optionally in combination with one or more features of the various embodiments described above or below, the halogenating agent is selected from the group consisting of phosphorus(V) oxychloride, thionyl chloride, phosphorus pentachloride, *N*-chlorosuccinimide, and trichloroisocyanuric acid, more particularly phosphorus(V) oxychloride, even more particularly the halogenating agent is used in an amount from 2 to 5 molar equivalents, more particularly about 2.6 molar equivalents, relative to the compound of formula (VII).

In another embodiment, optionally in combination with one or more features of the various embodiments described above or below, the sulfonylating agent is a sulfonyl halide of the formula R₁SO₂X, wherein R₁ is as defined herein and X is halogen, particularly chloro, even more particularly the sulfonylating agent is used in an amount from 2 to 5 molar equivalents, more particularly about 2.6 molar equivalents, relative to the compound of formula (VII).

In another embodiment, optionally in combination with one or more features of the various embodiments described above or below, the sulfonylating agent is a sulfonic acid anhydride of the formula R₁SO₂-O-SO₂R₁, wherein R₁ is as defined herein, even more particularly the sulfonylating agent is used in an amount from 2 to 5 molar equivalents, more particularly about 2.6 molar equivalents, relative to the compound of formula (VII).

In another embodiment, optionally in combination with one or more features of the various embodiments described above or below, the acylating agent is an acyl halide of the formula R₁COX, wherein R₁ is as defined herein and X is halogen, particularly chloro, even more particularly the acylating agent is used in an amount from 2 to 5 molar equivalents, more particularly about 2.6 molar equivalents, relative to the compound of formula (VII).

In another embodiment, optionally in combination with one or more features of the various embodiments described above or below, the acylating agent is an acid anhydride of the formula R₁CO-O-COR₁, wherein R₁ is as defined herein, even more particularly the acylating agent is used in an amount from 2 to 5 molar equivalents, more particularly about 2.6 molar equivalents, relative to the compound of formula (VII).

Step vi) may be performed in the presence of a base which is not particularly limited. The amount of the base may be generally from 1 to 3 molar equivalents, particularly about 1.8 molar equivalents, relative to compound of formula (VII). In one embodiment, optionally in combination with one or more features of the various embodiments described above or below, the base used in step vii) is selected from the group consisting of triethylamine, *N,N*-diisopropylethylamine (DIPEA), pyridine, N-methylmorpholine (NMM), *N-*methylimidazole (NMI), and combinations thereof, even more particularly the base is triethylamine.

Step vi) can be carried out in a suitable solvent such as organic solvent which is not particularly limited, and at a suitable temperature, e.g., by heating at a temperature from 60 to 65 °C, and with a reaction time generally from 2 to 18 h. In one embodiment, optionally in combination with one or more features of the various embodiments described above or below, step vi) is carried out in an organic solvent selected from the group consisting of *N,N*-dimethylformamide, *tert*-butanol, 1,4-dioxane, dichloromethane, dichloroethane, toluene, acetonitrile, tetrahydrofuran, 2-methyltetrahydrofuran, ethyl acetate, and combinations thereof, more particularly, the solvent is acetonitrile. According to one embodiment, optionally in combination with one or more features of the various embodiments described above or below, the invention relates to a process for the preparation of deucravacitinib of formula (XI) or a pharmaceutically acceptable salt thereof, which comprises:
vi) converting a compound of formula (VII), or a salt thereof, as defined herein, into compound of formula (VIII), or a salt thereof, as defined herein;
vii) reacting the compound of formula (VIII), or a salt thereof, with a compound of formula (VI), or a salt thereof as defined herein, to obtain a compound of formula (IX), or a salt thereof, as defined herein;
   a) reacting a compound of formula (IX), or a salt thereof, with methyl-*d*₃-amine, or a salt thereof, in the presence of cesium carbonate in dimethylformamide to give a compound of formula (X), or a salt thereof, as defined herein;
   b) reacting the compound of formula (X), or a salt thereof, with cyclopropanecarboxamide, or a salt thereof, to give a compound of formula (XI); and
   c) optionally converting deucravacitinib of formula (XI) into a pharmaceutically acceptable salt thereof.

In another embodiment, optionally in combination with one or more features of the various embodiments described above or below, in the process of the first aspect, previously to step vi), the process comprises:
v) reacting a compound of formula (V'), or a salt thereof, 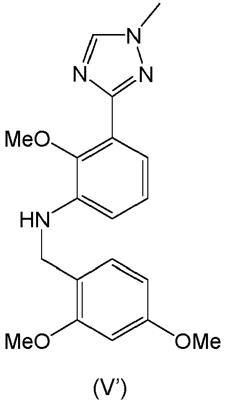 with an acid to give a compound of formula (VI), or a salt thereof; or alternatively
v') reacting a compound of formula (V"), or a salt thereof, 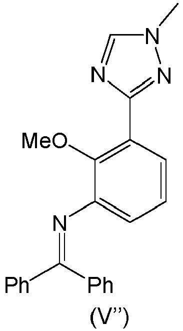
with an acid to give a compound of formula (VI), or a salt thereof.

Step v) can be carried out in the presence of a suitable acid which is not particularly limited. In one embodiment, optionally in combination with one or more features of the various embodiments described above or below, the acid is selected from the group consisting of trifluoroacetic acid, trifluoromethanesulfonic acid, methanesulfonic acid, *p-*toluenesulfonic acid, hydrochloric acid, and hydrobromic acid, more particularly trifluoroacetic acid, even more particularly the acid is used in an amount from 1 to 5 molar equivalents, more particularly about 2.5 molar equivalents, relative to the compound of formula (V').

Step v') can be carried out in the presence of a suitable acid which is not particularly limited. In one embodiment, optionally in combination with one or more features of the various embodiments described above or below, the acid is selected from the group consisting of trifluoroacetic acid, trifluoromethanesulfonic acid, methanesulfonic acid, *p-*toluenesulfonic acid, hydrochloric acid, and hydrobromic acid, more particularly hydrochloric acid, even more particularly the acid is used in an amount from 1 to 3 molar equivalents, more particularly about 1.8 molar equivalents, relative to the compound of formula (V').

Steps v) and v') can be carried out in a suitable solvent such as organic solvent which is not particularly limited, and at a suitable temperature, *e.g.,* at room temperature, and with a reaction time generally from 1 to 6 h. In one embodiment, optionally in combination with one or more features of the various embodiments described above or below, step v') is carried out in an organic solvent selected from the group consisting of *N,N-*dimethylformamide, *tert*-butanol, 1,4-dioxane, dichloromethane, dichloroethane, toluene, acetonitrile, tetrahydrofuran, 2-methyltetrahydrofuran, ethyl acetate, and combinations thereof, more particularly, the solvent is tetrahydrofuran.

In another embodiment, optionally in combination with one or more features of the various embodiments described above or below, in the process of the first aspect, previously to step v), the process comprises:
wherein previously to step v) the process comprises:
iv) reacting the compound of formula (IV), or a salt thereof, 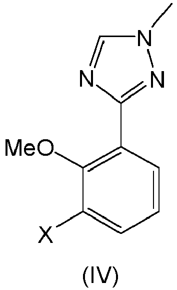 wherein X is halogen with 2,4-dimethoxybenzylamine. or a salt thereof, in presence of metallic catalyst to give a compound of formula (V'), or a salt thereof;
or alternatively previously to step v'), the process comprises:
   iv') reacting the compound of formula (IV), or a salt thereof, wherein X is halogen, with benzophenone imine, in presence of metallic catalyst to give a compound of formula (V") or a salt thereof.

Step iv) and iv') are carried out in the presence of a metallic catalyst, such a palladium catalyst, in an amount generally from 0.01 to 0.10 molar equivalents, particularly about 0.05 molar equivalents, relative to compound of formula (IV), and a phosphane ligand in an amount generally from 0.02 to 0.20 molar equivalents, particularly about 0.10 molar equivalents, relative to compound of formula (IV). In one embodiment, optionally in combination with one or more features of the various embodiments described above or below, step b) is carried out in the presence of a catalyst and a phosphane ligand, more particularly, the catalyst is selected from the group consisting of tris(dibenzylideneacetone)dipalladium(0) (Pd₂(dba)₃), Pd(OAc)₂, Pd(PPh₃)₂Cl₂, Pd(PPh₃)₄, Pd(dba)₂, and Pd(dppf)Cl₂, and the phosphane ligand selected from the group consisting of (±)-2,2'-bis(diphenylphosphino)-1,1'-binaphthalene (BINAP), of 1,1'-ferrocenediyl-bis(diphenylphosphine) (dppf), (2*R*)-1-[(1*R*)-1-[bis(1,1-dimethylethyl)phosphino]ethyl]-2-(dicyclohexylphosphino)ferrocene (Josiphos), 2-dicyclohexylphosphano-2'-(*N*,*N-*dimethylamino)biphenyl (DavePhos), and 4.5-bis(diphenylphosphano)-9,9-dimethylxanthene) (Xantphos), and even more particularly the catalyst is Pd(dba)₂ and the phosphane ligand is BINAP.

Steps iv) and iv') can be carried out in a suitable solvent such as organic solvent which is not particularly limited, and at a suitable temperature, e.g., by heating at a temperature from 80 to 120 °C, and with a reaction time generally from 1 to 6 h. In one embodiment, optionally in combination with one or more features of the various embodiments described above or below, step a) is carried out in an organic solvent selected from the group consisting of *N,N*-dimethylformamide, *tert*-butanol, 1,4-dioxane, dichloromethane, dichloroethane, toluene, acetonitrile, tetrahydrofuran, 2-methyltetrahydrofuran, ethyl acetate, and combinations thereof, more particularly, the solvent is toluene.

Steps iv) and iv') may be performed in the presence of a base which is not particularly limited. In one embodiment, optionally in combination with one or more features of the various embodiments described above or below, the base is selected from the group consisting of sodium tert-butoxide, potassium tert-butoxide, sodium isopropoxide, cesium carbonate, and potassium carbonate, more particularly sodium *tert*-butoxide, and even more particularly the base is used in an amount from 1 to 5 molar equivalents, particularly about 4 molar equivalents, relative to compound of formula (III).

Step iv) is carried out in the presence of 2,4-dimethoxybenzylamine or a salt thereof. In one embodiment, optionally in combination with one or more features of the various embodiments described above or below, the nucleophile (amine) is used in an amount from 1 to 3 molar equivalents, more particularly about 1.5 molar equivalents, relative to the compound of formula (IV).

Step iv') is carried out in the presence of benzophenone imine or a salt thereof. In one embodiment, optionally in combination with one or more features of the various embodiments described above or below, the nucleophile (imine) is used in an amount from 1 to 3 molar equivalents, more particularly about 1.5 molar equivalents, relative to the compound of formula (V').

According to one embodiment, optionally in combination with one or more features of the various embodiments described above or below, in the compound of formula (IV), X is bromo.

In another embodiment, optionally in combination with one or more features of the various embodiments described above or below, in the process of the first aspect, previously to step iv) or alternatively step iv') the process comprises:
iii) reacting the compound of formula (III), or a salt thereof, 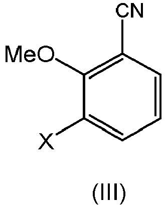 wherein X is halogen, with *N*-formyl-*N*-methylhydrazine, or a salt thereof, to give a compound of formula (IV), or a salt thereof.

Step iii) is carried out in the presence of *N*-formyl-*N*-methylhydrazine or a salt thereof. In one embodiment, optionally in combination with one or more features of the various embodiments described above or below, the hydrazinde derivative is used in an amount from 1 to 3 molar equivalents, more particularly about 1.8 molar equivalents, relative to the compound of formula (III).

Step iii) may be performed in the presence of a base which is not particularly limited. In one embodiment, optionally in combination with one or more features of the various embodiments described above or below, the base is selected from the group consisting of potassium *tert*-butoxide, sodium tert-butoxide, sodium isopropoxide, cesium carbonate, and potassium carbonate, more particularly potassium *tert*-butoxide, and even more particularly the base is used in an amount from 1 to 5 molar equivalents, particularly about 2.4 molar equivalents, relative to compound of formula (III).

Step iii) can be carried out in a suitable solvent such as organic solvent which is not particularly limited, and at a suitable temperature, *e.g.,* by cooling at a temperature from 0 to 10 °C, and with a reaction time generally from 1 to 4 h. In one embodiment, optionally in combination with one or more features of the various embodiments described above or below, step iii) is carried out in an organic solvent selected from the group consisting of *N,N*-dimethylformamide, *tert*-butanol, 1,4-dioxane, dichloromethane, dichloroethane, toluene, acetonitrile, tetrahydrofuran, 2-methyltetrahydrofuran, ethyl acetate, and combinations thereof, more particularly, the solvent is tetrahydrofuran.

In another embodiment, optionally in combination with one or more features of the various embodiments described above or below, in the process of the first aspect, previously to step iii) the process comprises:
ii) converting a compound of formula (II), or a salt thereof, 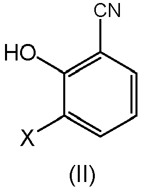 wherein X is halogen; into the compound of formula (III), or a salt thereof.

Step ii) may be carried out in the presence of an alkylating agent which is not particularly limited. In one embodiment, optionally in combination with one or more features of the various embodiments described above or below, the alkylating agent is selected from the group consisting of iodomethane, bromomethane, chloromethane, and dimethyl sulfate, more particularly iodomethane, and even more particularly the alkylating agent is used in an amount from 1 to 3 molar equivalents, more particularly about 1.5 molar equivalents, relative to the compound of formula (II).

Step ii) may be performed in the presence of a base which is not particularly limited. The amount of the base may be generally from 1 to 5 molar equivalents, particularly about 2 molar equivalents, relative to compound of formula (II). In one embodiment, optionally in combination with one or more features of the various embodiments described above or below, the base used in step ii) is selected from the group consisting of sodium carbonate, sodium hydrogen carbonate, potassium carbonate, potassium hydrogen carbonate, potassium phosphate, cesium carbonate, and combinations thereof, more particularly, the base is potassium carbonate.

In another embodiment, optionally in combination with one or more features of the various embodiments described above or below, the base is selected from the group consisting of potassium carbonate, cesium carbonate, potassium *tert*-butoxide, sodium *tert*-butoxide, sodium isopropoxide, more particularly potassium carbonate, and even more particularly the base is used in an amount from 1 to 5 molar equivalents, particularly about 2.0 molar equivalents, relative to compound of formula (II).

Step ii) can be carried out in a suitable solvent such as organic solvent which is not particularly limited, and at a suitable temperature, *e.g.,* at room temperature, and with a reaction time generally from 2 to 6 h. In one embodiment, optionally in combination with one or more features of the various embodiments described above or below, step ii) is carried out in an organic solvent selected from the group consisting of *N,N-*dimethylformamide, tert-butanol, 1,4-dioxane, dichloromethane, dichloroethane, toluene, acetonitrile, tetrahydrofuran, 2-methyltetrahydrofuran, ethyl acetate, and combinations thereof, more particularly, the solvent is *N*,*N*-dimethylformamide.

In another embodiment, optionally in combination with one or more features of the various embodiments described above or below, in the process of the first aspect, previously to step ii) the process comprises:
i) halogenating a compound of formula (I), or a salt thereof, 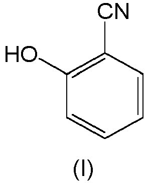 to give a compound of formula (II), or a salt thereof.

Step i) may be carried out in the presence of a halogenating agent. In one embodiment, optionally in combination with one or more features of the various embodiments described above or below, the halogenating agent is selected from the group consisting of *N-*bromosuccinimide (NBS), bromine, *N*-bromophthalimide, N-iodosuccinimide, *N-*chlorosuccinimide (NCS), *N*-chlorophthalimide (NCP), trifluoromethanesulfonyl chloride, and 1,3-dichloro-5,5-dimethylhydantoin, more particularly the halogenating agent is bromine, even more particularly the halogenating agent used in an amount from 1 to 2 molar equivalents, more particularly about 1.1 molar equivalents, relative to the compound of formula (I).

Step i) may be performed in the presence of a base which is not particularly limited. The amount of the base may be generally from 1 to 5 molar equivalents, particularly about 3 molar equivalents, relative to compound of formula (I). In one embodiment, optionally in combination with one or more features of the various embodiments described above or below, the base used in step i) is selected from the group consisting of *tert*-butylamine, triethylamine, *N*,*N*-diisopropylethylamine, pyridine, *N*-methylmorpholine, and combinations thereof, even more particularly the base is *tert*-butylamine.

Step i) can be carried out in a suitable solvent such as organic solvent which is not particularly limited, and at a suitable temperature, *e.g.,* at room temperature, and with a reaction time generally from 10 to 24 h. In one embodiment, optionally in combination with one or more features of the various embodiments described above or below, step i) is carried out in an organic solvent selected from the group consisting of *N,N-*dimethylformamide, *tert*-butanol, 1,4-dioxane, dichloromethane, dichloroethane, toluene, acetonitrile, tetrahydrofuran, 2-methyltetrahydrofuran, ethyl acetate, and combinations thereof, more particularly, the solvent is a mixture of toluene and dichloromethane.

In another embodiment, optionally in combination with one or more features of the various embodiments described above or below, the invention relates to a process for the preparation of deucravacitinib of formula (XI) or a pharmaceutically acceptable salt thereof, which comprises:
i) halogenating a compound of formula (I), or a salt thereof, as defined herein to give a compound of formula (II), or a salt thereof, as defined herein;
ii) converting the compound of formula (II), or a salt thereof, into the compound of formula (III), or a salt thereof, as defined herein;
iii) reacting the compound of formula (III), or a salt thereof, with *N*-formyl-*N-*methylhydrazine, or a salt thereof, to obtain a compound of formula (IV), or a salt thereof, as defined herein;
iv) reacting the compound of formula (IV), or a salt thereof, with 2,4-dimethoxybenzylamine or a salt thereof, in presence of metallic catalyst, to obtain the compound of formula (V'), or a salt thereof, as defined herein; and
v) reacting the compound of formula (V'), or a salt thereof with an acid to give a compound of formula (VI), or a salt thereof, as defined herein; or alternatively or alternatively
   iv') reacting the compound of formula (IV), or a salt thereof, with benzophenone imine, in presence of metallic catalyst, to obtain the compound of formula (V"), or a salt thereof, as defined herein; and
   v') reacting the compound of formula (V"), or a salt thereof with an acid to give a compound of formula (VI), or a salt thereof, as defined herein;
vi) converting a compound of formula (VII), or a salt thereof, as defined herein, into compound of formula (VIII), or a salt thereof, as defined herein;
vii) reacting the compound of formula (VIII), or a salt thereof, with the compound of formula (VI), or a salt thereof, to obtain a compound of formula (IX), or a salt thereof, as defined herein;
   a) reacting a compound of formula (IX), or a salt thereof, with methyl-*d*₃-amine, or a salt thereof, in the presence of cesium carbonate in dimethylformamide to give a compound of formula (X), or a salt thereof, as defined herein;
   b) reacting the compound of formula (X), or a salt thereof, with cyclopropanecarboxamide, or a salt thereof, to give a compound of formula (XI); and
   c) optionally converting deucravacitinib of formula (XI) into a pharmaceutically acceptable salt thereof.

As mentioned above the present invention also relates to the intermediates of formula (V') and (V") used in the processes disclosed herein.

As mentioned above, a second aspect of the invention relates to a process for the preparation of a compound of formula (V'), or a salt thereof as defined herein, which comprises:
iv) reacting the compound of formula (IV), or a salt thereof as defined herein, with 2,4-dimethoxybenzylamine or a salt thereof, in presence of metallic catalyst.

In one embodiment, optionally in combination with one or more features of the various embodiments described above or below, in the process of the second aspect, previously to step iv) the process comprises:
iii) reacting the compound of formula (III), or a salt thereof as defined herein, with *N-*formyl-*N*-methylhydrazine, or a salt thereof, to give a compound of formula (IV), or a salt thereof as defined herein.

In another embodiment, optionally in combination with one or more features of the various embodiments described above or below, in the process of the second aspect, previously to step iii) the process comprises:
ii) converting a compound of formula (II), or a salt thereof as defined herein; into the compound of formula (III), or a salt thereof as defined herein.

In another embodiment, optionally in combination with one or more features of the various embodiments described above or below, in the process of the second aspect, previously to step iii) the process comprises:
i) halogenating a compound of formula (I), or a salt thereof as defined herein, to give a compound of formula (II), or a salt thereof as defined herein.

In another embodiment, optionally in combination with one or more features of the various embodiments described above or below, the process of the second aspect comprises:
i) halogenating a compound of formula (I), or a salt thereof, as defined herein to give a compound of formula (II), or a salt thereof, as defined herein;
ii) converting the compound of formula (II), or a salt thereof, into the compound of formula (III), or a salt thereof, as defined herein;
iii) reacting the compound of formula (III), or a salt thereof, with *N*-formyl-*N-*methylhydrazine, or a salt thereof, to obtain a compound of formula (IV), or a salt thereof, as defined herein; and
iv') reacting the compound of formula (IV), or a salt thereof, with benzophenone imine, in presence of metallic catalyst.

The third aspect of the invention relates to a process for the preparation of a compound of formula (V"), or a salt thereof as defined herein, which comprises:
iv') reacting the compound of formula (IV), or a salt thereof, with benzophenone imine, in presence of metallic catalyst.

In one embodiment, optionally in combination with one or more features of the various embodiments described above or below, in the process of the third aspect, previously to step iv) the process comprises:
iii) reacting the compound of formula (III), or a salt thereof as defined herein, with *N-*formyl-*N*-methylhydrazine, or a salt thereof, to give a compound of formula (IV), or a salt thereof as defined herein.

In another embodiment, optionally in combination with one or more features of the various embodiments described above or below, in the process of the third aspect, previously to step iii) the process comprises:
ii) converting a compound of formula (II), or a salt thereof as defined herein; into the compound of formula (III), or a salt thereof as defined herein.

In another embodiment, optionally in combination with one or more features of the various embodiments described above or below, in the process of the third aspect, previously to step iii) the process comprises:
i) halogenating a compound of formula (I), or a salt thereof as defined herein, to give a compound of formula (II), or a salt thereof as defined herein.

In another embodiment, optionally in combination with one or more features of the various embodiments described above or below, the process of the third aspect comprises:
i) halogenating a compound of formula (I), or a salt thereof, as defined herein to give a compound of formula (II), or a salt thereof, as defined herein;
ii) converting the compound of formula (II), or a salt thereof, into the compound of formula (III), or a salt thereof, as defined herein;
iii) reacting the compound of formula (III), or a salt thereof, with *N*-formyl-*N-*methylhydrazine, or a salt thereof, to obtain a compound of formula (IV), or a salt thereof, as defined herein; and
iv) reacting the compound of formula (IV), or a salt thereof, with 2,4-dimethoxybenzylamine or a salt thereof, in presence of metallic catalyst.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

NMR spectra were acquired on a Bruker AVANCE NEO NanoBay instrument operating at 400 MHz for ¹H NMR, using the residual solvent signal as the internal standard and with recording at 300 K in all cases. Chemical shifts (*δ*) are expressed in ppm and coupling constants (J) in Hertz. The following abbreviations are used: bs, broad singlet; s, singlet; bd, broad doublet; dd, double of doublets; m, multiplet.

High Performance Liquid Chromatography (HPLC) analyses were performed using a Waters e2695 HPLC instrument equipped with Waters 2489 UV/PDA detector using Waters X-Bridge C18 chromatographic column (4.6 x 150 mm, 3.5 µm) under following method conditions:
- Column temperature: 25 °C
- Wavelength: 215 nm
- Injection volume: 4.0 µL
- Flow rate: 1.0 mL/min
- Mobile Phase A: 0.1% orthophosphoric acid (OPA) in water
- Mobile Phase B: 0.1% orthophosphoric acid (OPA) in 95:5 (v/v) acetonitrile/water
- Diluent: 50:50 (v/v) water/acetonitrile (0.1% OPA in water/acetonitrile for reaction samples making use of potassium carbonate)
- Gradient table:

| Time (min) | % Mobile Phase A | % Mobile Phase B |
|---|---|---|
| 0.0 | 95 | 5 |
| 2 | 95 | 5 |
| 13 | 5 | 95 |
| 20 | 5 | 95 |
| 20.1 | 95 | 5 |
| 25 | 95 | 5 |

Mass spectra were obtained by LC-MS using the electrospray ionization mode (ESI) under positive or negative polarity.

### Example 1. Preparation of 3-bromo-2-hydroxybenzonitrile (II)

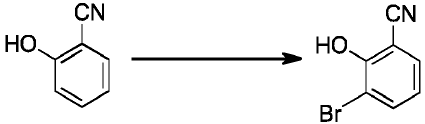

Tert-butylamine (26.4 mL, 251.84 mmol) was charged to a three-necked 500 mL Round Bottom Flask (RBF) at 25 °C. Toluene (200 mL) was added, and the resulting mixture was cooled to -30 °C. Bromine (4.7 mL, 92.34 mmol) was then loaded slowly over the previous mixture, which was further cooled down to -78 °C. Commercially available 2-hydroxybenzonitrile (I; 10 g, 83.95 mmol; HPLC purity: >99% a/a; HPLC RT: 9.4 min) was added to the reaction as a suspension in DCM (50 mL). The reaction mixture was warmed to 25 °C and further stirred overnight. After that, the mixture was diluted with brine (500 mL). The layers were separated, and the aqueous layer was further extracted with ethyl acetate (500 mL). The combined organic layers were treated with 10% aq. NaOH solution (2 x 500 mL), and the phases were separated. The combined aqueous layers (containing product as a sodium salt) were acidified with 6 N HCl (450 mL) up to pH 1-2 and further extracted using DCM (2 x 500 mL). The final organic layer was dried over Na₂SO₄ and concentrated to dryness under reduced pressure to render 3-bromo-2-hydroxybenzonitrile (II) as an off-white solid (12 g, 72% yield; HPLC purity: 87% a/a; HPLC RT: 10.6 min). ¹H NMR (400 MHz, MeOH-*d*₄) *δ* 6.91 (dd, *J* = 8.0 and 8.0, 1H, *H*_{Ar}), 7.56 (dd, *J* = 8.0 and 1.6, 1H, *H*_{Ar}), 7.79 (dd, *J* = 8.0 and 1.6, 1H, *H*_{Ar}); MS (ESI-): *m*/*z* 196.17/198.17 ⁷⁹Br/⁸¹Br [M-H]-.

### Example 2. Preparation of 3-bromo-2-methoxybenzonitrile (III)

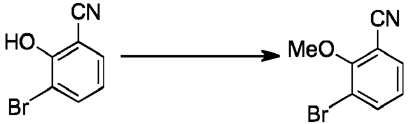

3-Bromo-2-hydroxybenzonitrile (II; 1.0 g, 5.05 mmol) was charged to a two-necked 25 mL RBF under nitrogen atmosphere at 25 °C. DMF (10 mL) followed by K₂CO₃ (1.39 g, 10.10 mmol) was added, and the resulting mixture was stirred for 20 min before iodomethane (0.47 mL, 7.58 mmol) addition dropwise for 10 min. The reaction mixture was stirred at 25 °C for 4 h. After that, the mixture was diluted with cold water (40 mL). The resulting suspension was stirred at 0-5 °C for 30 min and then, filtered off. The wet cake was washed using cold water (2 mL) and dried under vacuum to give 3-bromo-2-methoxybenzonitrile (III) as an off-white solid (0.96 g, 90% yield; HPLC purity: 96% a/a; HPLC RT: 12.5 min). ¹H NMR (400 MHz, DMSO-*d*₆) *δ* 4.08 (s, 3H, OCH₃), 7.51 (dd, *J* = 8.0 and 8.0, 1H, *H*_{Ar}), 8.19 (dd, *J* = 8.0 and 1.6, 1H, *H*_{Ar}), 8.28 (dd, *J* = 8.0 and 1.6, 1H, *H*Ar).

### Example 3. Preparation of 3-(3-bromo-2-methoxyphenyl)-1-methyl-1H-1,2,4-triazole oxalate (IV)

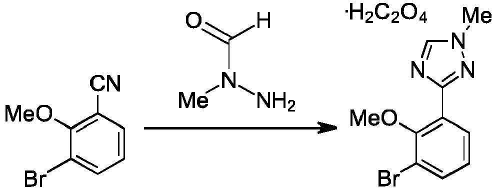

Potassium tert-butoxide (12.7 g, 56.59 mmol) was charged to a three-necked 250 mL RBF at 25 °C. Tetrahydrofuran (30 mL) was added, and the resulting mixture was cooled to 0 °C. A solution of 3-bromo-2-methoxybenzonitrile (III; 5.0 g, 23.58 mmol) and *N*-formyl-*N-*methylhydrazine (3.14 g, 42.44 mmol) in THF (20 mL) was added dropwise for 15 min. The reaction mixture was cooled to 0-10 °C and further stirred at this temperature for 1.5 h. After that, the mixture was quenched with aqueous NH₄Cl solution (50 mL), and layers were separated at 25 °C. The aqueous layer was extracted with EtOAc (2 x 50 mL). The combined organic layers were washed with water (25 mL) and subsequently with brine (25 mL). The resulting organic layer was further treated with a solution of oxalic acid (4.24 g, 47.16 mmol) in methanol (21.2 mL). The thus obtained mixture was heated to 50-55 °C and stirred at this temperature for 1 h. The resulting solution was then gradually cooled down to 0-5 °C for 2 h. Solvent was largely removed under reduced procedure (final volume approx. 5 mL). The resulting residue was diluted with EtOAc (25 mL), stirred for 10 min at 25-30 °C and further stirred at 5-10 °C for 1 h. The slurry was filtered, and the wet cake was washed using cold EtOAc (5 mL) and dried under vacuum to render 3-(3-bromo-2-methoxyphenyl)-1-methyl-1*H*-1,2,4-triazole oxalate (IV) as a light brown solid (3.5 g, 42% yield; HPLC purity: 91% a/a; HPLC RT: 10.5 min). ¹H NMR (400 MHz, DMSO-*d*₆) *δ* 3.79 (s, 3H, NC*H*₃), 3.95 (s, 3H, OCH₃), 7.16 (dd, *J* = 7.6 and 7.6, 1H, *H*_{Ar}, phenyl), 7.70 (dd, *J* = 7.6 and 1.6, 1H, *H*_{Ar}, phenyl), 7.84 (dd, *J* = 7.6 and 1.6, 1H, *H*_{Ar}, phenyl), 8.57 (s, 1H, *H*_{Ar}, triazolyl); MS (ESI+): *m*/*z* 268.21/270.25 ⁷⁹Br/⁸¹Br [M + H]⁺.

### Example 4. Preparation of N-(diphenylmethylidene)-[2-methoxy-3-(1-methyl-1H-1,2,4-triazol-3-yl)phenyl7amine (V")

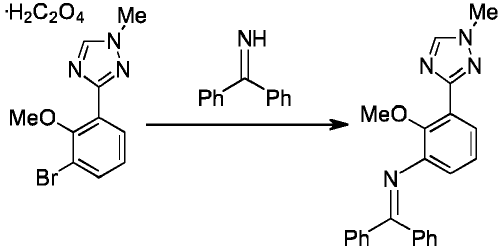

3-(3-Bromo-2-methoxyphenyl)-1-methyl-1*H*-1,2,4-triazole oxalate (IV; 2.0 g, 5.602 mmol) was charged to a three-necked 50 mL RBF under nitrogen atmosphere at 25 °C. Toluene (20 mL) was added, followed by loading of benzophenone imine (1.52 g, 8.403 mmol) and sodium tert-butoxide (2.15 g, 22.40 mmol) added subsequently at 25 °C. The resulting mixture was purged under nitrogen stream for 20 min. Then, tris(dibenzylideneacetone)-dipalladium(0) (Pd₂(dba)₃, 0.256 g, 0.280 mmol) and (±)-2,2'-bis(diphenylphosphino)-1,1'-binaphthalene (BINAP, 0.348 g, 0.560 mmol) were added under nitrogen atmosphere over the previous suspension at 25 °C. The reaction mixture was heated to 110-115 °C and further stirred at this temperature for 3 h. After that, the mixture was cooled down to 25 °C and filtered through a Celite pad, which was washed with EtOAc (20 mL). The filtrate was concentrated to dryness under reduced pressure to yield crude *N*-(diphenylmethylidene)-[2-methoxy-3-(1-methyl-1*H*-1,2,4-triazol-3-yl)phenyl]amine (V") as a yellowish solid (unstable compound under HPLC conditions). This crude product was used in the next step without further purification. ¹H NMR (400 MHz, DMSO-*d*₆) *δ* 3.77 (s, 3H, NC*H*₃), 3.90 (s, 3H, OCH₃), 6.70 (dd, *J* = 7.6 and 1.6, 1H, *H*_{Ar}, phenyl), 6.92 (dd, *J* = 7.6 and 7.6, 1H, *H*_{Ar}, phenyl), 7.14-7.20 (m, 2*H*_{Ar}, phenyl), 7.26-7.36 (m, 4*H*_{Ar}, phenyl), 7.47-7.53 (m, 2*H*_{Ar}, phenyl), 7.53-7.57 (m, 1*H*_{Ar}, phenyl), 7.68-7.74 (m, 2*H*_{Ar}, phenyl), 8.47 (s, 1H, *H*_{Ar}, triazolyl); MS (ESI+): *m*/*z* 369.49 [M + H]⁺.

### Example 5. Preparation of 2-methoxy-3-(1-methyl-1H-1,2,4-triazol-3-yl)aniline (VI)

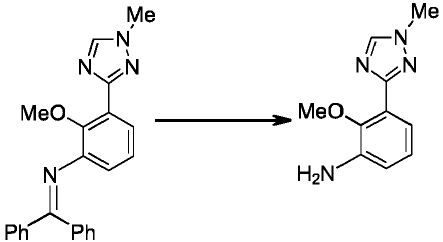

Crude *N*-(diphenylmethylidene)-[2-methoxy-3-(1-methyl-1*H*-1,2,4-triazol-3-yl)phenyl]amine (V"; 5.602 mmol) was charged to three-necked 25 mL RBF at 25 °C. Tetrahydrofuran (15 mL) was loaded at 25 °C, followed by dropwise addition of aqueous 1.0 N HCI solution (0.3 mL, 10.14 mmol). The reaction mixture was stirred at 25 °C for 1 h. After that, the solvent was removed under reduced pressure to give a residue which was diluted with water (2 mL) and further washed with DCM (2 x 15 mL). The aqueous layer was basified with 2.0 N NaOH until pH 8-9 and then, extracted with DCM (2 x 30 mL). The combined organic layers were concentrated *in vacuo* yielding an residue, which was purified by silica gel column chromatography (eluent: gradient of hexanes/EtOAc) to render 2-methoxy-3-(1-methyl-1*H*-1,2,4-triazol-3-yl)aniline (VI) as an off-white solid (0.74 g, 65% overall yield after two steps; HPLC purity: >99% a/a; HPLC RT: 4.8 min). ¹H NMR (400 MHz, DMSO-*d*₆) *δ* 3.66 (s, 3H, NC*H*₃), 3.91 (s, 3H, OC*H*₃), 4.96 (bs, 2H, N*H*₂), 6.74 (dd, *J* = 8.0 and 2.0, 1H, *H*_{Ar}, phenyl), 6.85 (dd, *J* = 8.0 and 8.0, 1H, *H*_{Ar}, phenyl), 6.95 (dd, *J* = 8.0 and 2.0, 1H, *H*_{Ar}, phenyl), 8.46 (s, 1H, *H*_{Ar}, triazolyl); MS (ESI+): *m*/*z* 205.13 [M + H]⁺.

### Example 6. Telescoped preparation of methyl 4,6-dihydroxypyridazine-3-carboxylate (VII)

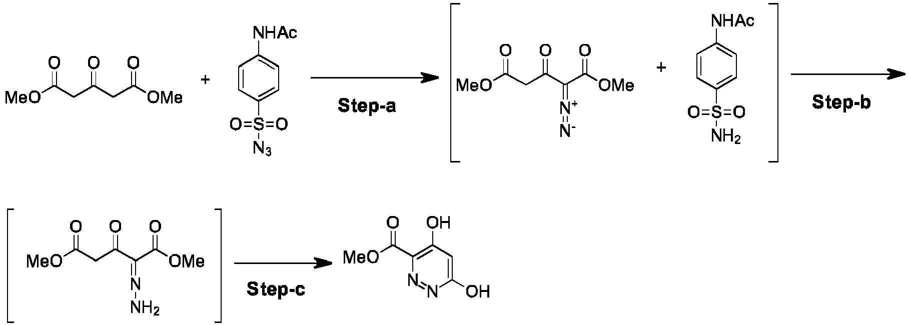

### Step-a (diazo transfer reaction):

Commercially available dimethyl-1,3-acetonedicarboxylate (86.98 g, 499.50 mmol; HPLC purity: >99% a/a; HPLC RT: 8.9 min) was charged to a three-necked 2 L RBF at 25 °C. Acetonitrile (600 mL) was loaded at 25 °C, followed by addition of *N*,*N*-diisopropylethylamine (3.6 mL, 20.81 mmol). The resulting mixture was cooled to 0-5 °C and then, a solution of commercially available 4-acetamidobenzene sulfonylazide (100.0 g, 416.25 mmol; HPLC purity: >99% a/a; HPLC RT: 10.6 min) in acetonitrile (500 mL) was slowly added for 5 min. The reaction mixture was stirred at 0-5 °C for 1 h. After that, the thus obtained slurry was filtered at 25 °C and the filter cake was washed gently with acetonitrile (200 mL). The solid filter cake containing 4-acetamidobenzenesulfonamide (HPLC RT: 6.5 min) as the reaction byproduct was discarded. The filtrate as a yellowish solution of non-isolated dimethyl 2-diazo-3-oxopentanedioate in acetonitrile was directly used for Step-b as is.

### Steps-b & c (reduction & cyclization reactions):

The filtrate coming from Step-a was transferred to a suitable three-necked RBF at 25 °C and then, cooled to 0-5 °C. Water (16.5 mL, 915.75 mmol) and tri-n-butylphosphine (112.9 mL, 457.88 mmol) were sequentially loaded over the solution at 0-5 °C. The reaction mixture was stirred at 0-5 °C for 2 h, with formation of non-isolated dimethyl 2-hydrazono-3-oxopentanedioate (HPLC RT: 6.3 min) confirmed by HPLC. Subsequently, acetic acid (48.8 mL, 853.31 mmol) was added to the mixture at 0-5 °C. The resulting reaction mixture was heated to 75 °C and further stirred at this temperature for 6 h. After this period, the mixture was cooled to 45 °C and then, concentrated partially under reduced pressure (final volume approx. 300 mL). The thus obtained suspension was cooled to 10-15 °C and stirred at this temperature for 3 h. The slurry was filtered, and the wet cake was washed with acetonitrile (100 mL) and dried under vacuum to render methyl 4,6-dihydroxypyridazine-3-carboxylate (VII) as an off-white solid (29.0 g, 41% overall yield after three steps; HPLC purity: 98% a/a; HPLC RT: 6.2 min). ¹H NMR (400 MHz, DMSO-*d*₆) *δ* 3.82 (s, 3H, OC*H*₃), 6.02 (s, 1H, *H*_{Ar}), 11.6 (bs, 1H, O*H*), 13.0 (bs, 1H, O*H*); MS (ESI+): *m*/*z* 171.02 [M + H]⁺.

### Example 7. Preparation of methyl 4,6-dichloropyridazine-3-carboxylate (Villa)

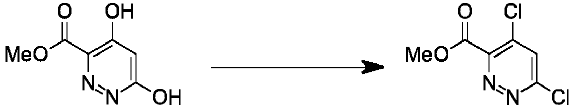

Acetonitrile (1.5 L) and methyl 4,6-dihydroxypyridazine-3-carboxylate (VII; 150.0 g, 0.882 mol) were charged under nitrogen atmosphere into a 5 L glass-jacketed reactor at 25-30 °C. The resulting stirred solution was cooled to 0-5 °C under nitrogen atmosphere. Phosphorus(V) oxychloride (214 mL, 2.29 mol) was added dropwise for about 20 min over the previous mixture cooled at 0-5 °C, followed by dropwise addition of triethylamine (228 mL, 1.63 mol) for about 50 min, maintaining the internal temperature below 10 °C (note: exothermic addition). The resulting mixture was heated to 60-65 °C and further stirred at this temperature under nitrogen atmosphere for 7 h. Afterwards, the reaction mixture was slowly cooled down to 0-5 °C, stirred for 1 h, and then, quenched with water (0.3 L) followed by pH 7 buffer solution (1.2 L) [pH 7 buffer solution: K₂HPO₄ (462 g, 2.65 mol) was dissolved in water (3.6 L) and adjusted to pH 7 using 1.0 N HCI], maintaining the internal temperature below 5 °C. The product was subsequently extracted with methyl *tert-*butyl ether (MTBE, 2.1 L). The separated organic layer at 0-5 °C was washed twice with pH 7 buffer solution (2 x 1.2 L) and then, twice with water (2 x 0.75 L). The combined aqueous layers were further extracted with MTBE (0.45 L). The finally combined organic layers were first concentrated under reduced pressure at 40-45 °C as the jacket temperature (final volume approx. 0.15-0.30 L). After n-heptane (0.75 L) addition, the resulting solution was subjected to a second vacuum distillation at 50-55 °C as the jacket temperature (final volume approx. 0.15-0.30 L). The thus obtained thick solution was further diluted with *n*-heptane (1.2 L), stirred at 25-30 °C for 30 min followed by cooling down at 0-5 °C, and stirring under this temperature for 30 min. The slurry was then filtered, with the wet cake washed with cold *n*-heptane (150 mL) and dried under vacuum to render methyl 4,6-dichloropyridazine-3-carboxylate (Vllla) as a light brown solid ¹H NMR (400 MHz, DMSO-*d*₆) *δ* 4.00 (s, 3H, OC*H*₃), 8.58 (s, 1H, *H*_{Ar}); MS (ESI+): *m*/*z* 207.05/209.22/211.04 ³⁵Cl³⁵Cl/³⁵Cl³⁷Cl/³⁷Cl³⁷Cl [M + H]⁺.

### Example 8. Preparation of methyl 6-chloro-4-((2-methoxy-3-(1-methyl-1H-1,2,4-triazol-3-yl)phenyl)amino)pyridazine-3-carboxylate (IXa)

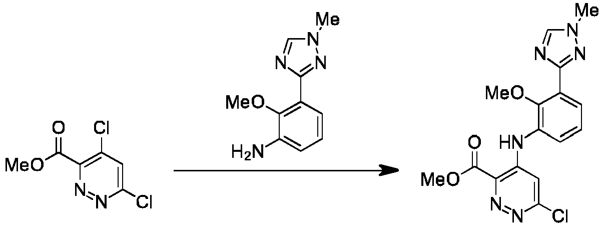

*Tert*-butanol (2.2 L), 2-methoxy-3-(1-methyl-1*H*-1,2,4-triazol-3-yl)aniline (VI; 220.0 g, 1.08 mol) and methyl 4,6-dichloropyridazine-3-carboxylate (Villa, 289.9 g, 1.40 mol) were charged under nitrogen atmosphere into a 20 L multi-necked RBF at 25-30 °C. *N,N-*diisopropylethylamine (282 mL, 1.62 mol) was added at 25-30 °C over the previously prepared stirred solution under nitrogen atmosphere. The reaction mixture was heated to 80-85 °C (reflux) and further stirred at this temperature under nitrogen atmosphere for 90 h. Afterwards, the resulting suspension was cooled down to 25-30 °C and diluted with DCM (4.4 L). The thus obtained solution was treated with aqueous NH₄Cl solution (2.2 L), with stirring at 25-30 °C for around 15 min, followed by phase separation. The separated aqueous layer was back extracted with DCM (1.1 L). The combined organic layers were washed with water (1.1 L). The final organic layer was first concentrated under reduced pressure (final volume approx. 0.44 L), followed by solvent swap twice with isopropyl acetate (IPAc, 2 x 1.1 L) and subsequent distillations (each final volume approx. 0.44 L). The thus obtained slurry was further diluted with IPAc (1.1 L), stirred at 25-30 °C for 30 min followed by cooling down at 0-5 °C, and stirring under this temperature for 30 min. The slurry was then filtered, with the wet cake washed thrice with cold IPAc (3 x 0.66 L) and dried under vacuum at 50 °C for 6 h to yield methyl 6-chloro-4-((2-methoxy-3-(1-methyl-1*H*-1,2,4-triazol-3-yl)phenyl)amino)pyridazine-3-carboxylate (IXa) as a light brown solid (277.0 g, 69% yield; HPLC purity: >98% a/a; HPLC RT: 9.0 min). ¹H NMR (400 MHz, DMSO-*d*₆) *δ* 3.70 (s, 3H, NC*H*₃), 3.95 (s, 3H, OCH₃), 3.99 (s, 3H, OCH₃), 7.07 (s, 1H, *H*_{Ar}, pyridazinyl), 7.31 (dd, *J* = 7.6 and 7.6, 1H, *H*_{Ar}, phenyl), 7.56 (dd, *J* = 7.6 and 1.6, 1H, *H*_{Ar}, phenyl), 7.77 (dd, *J* = 7.6 and 1.6, 1H, *H*_{Ar}, phenyl), 8.57 (s, 1H, *H*_{Ar}, triazolyl), 9.79 (bs, 1H, NH); MS (ESI+): *m*/*z* 375.28/377.41 ³⁵Cl/³⁵Cl [M + H]⁺.

### Example 9. Preparation of 6-chloro-4-((2-methoxy-3-(1-methyl-1H-1,2,4-triazol-3-yl)phenyl)amino)-N-(trideuteromethyl)pyridazine-3-carboxamide (Xa)

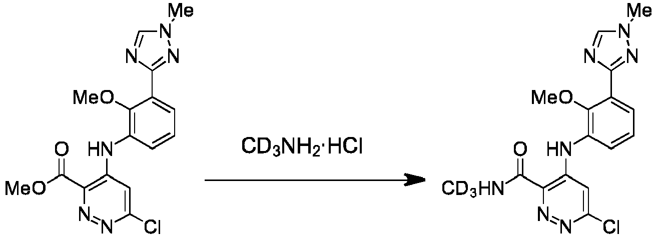

*N*,*N*-Dimethylformamide (3.5 L) and methyl 6-chloro-4-((2-methoxy-3-(1-methyl-1*H*-1,2,4-triazol-3-yl)phenyl)amino)pyridazine-3-carboxylate (IXa; 270.0 g, 0.72 mol) were charged under nitrogen atmosphere into a 20 L multi-necked RBF at 25-30 °C. Trideuteromethylamine hydrochloride (127.0 g, 1.80 mol) was added at 25-30 °C over the previously prepared stirred solution under nitrogen atmosphere. The resulting solution was cooled to 0-5 °C, and then, cesium carbonate (563.3 g, 1.73 mol) was loaded over the cold and stirred solution under nitrogen atmosphere. The reaction mixture was allowed to warm to 25-30 °C over a period of 1.5 h, and further stirred under such conditions for 20 h. Afterwards, the resulting suspension was cooled down to 15-20 °C and then, water (7.0 L) was slowly added for 30 min (note: exothermic addition). The thus obtained slurry was cooled down at 0-5 °C, and further stirred under this temperature for 1 h. The slurry was then filtered, with the wet cake washed twice with water (2 x 2.7 L) and dried under vacuum at 60 °C for 12 h to yield 6-chloro-4-((2-methoxy-3-(1-methyl-1*H*-1,2,4-triazol-3-*yl)phenyl)amino)-N-(trideuteromethyl)pyridazine-3-carboxamide* (Xa) as an off-white solid (229.0 g, 84.3% yield; HPLC purity: >99% a/a; HPLC RT: 9.3 min). ¹H NMR (400 MHz, DMSO-*d*₆) *δ* 3.72 (s, 3H, NC*H*₃), 3.95 (s, 3H, OCH₃), 7.19 (s, 1H, *H*_{Ar}, pyridazinyl), 7.29 (dd, *J* = 7.6 and 7.6, 1H, *H*_{Ar}, phenyl), 7.60 (bd, *J* = 7.6, 1H, *H*_{Ar}, phenyl), 7.72 (dd, *J* = 7.6 and 1.2, 1H, *H*_{Ar}, phenyl), 8.57 (s, 1H, *H*_{Ar}, triazolyl), 9.36 (bs, 1H, N*H*, amine), 11.1 (bs, 1H, N*H*, amide); MS (ESI+): *m*/*z* 377.40/379.39 ³⁵Cl/³⁵Cl [M + H]⁺.

### Example 10. Preparation of 6-(cyclopropanecarboxamido)-4-((2-methoxy-3-(1-methyl-1H-1,2,4-triazol-3-yl)phenyl)amino)-N-(trideuteromethyl)pyridazine-3-carboxamide [Deucravacitinib Form A]

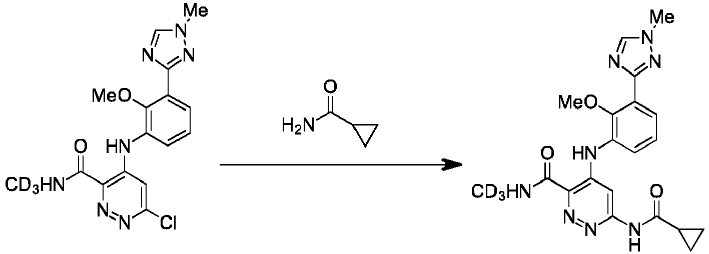

### Synthesis of crude deucravacitinib (cross-coupling step):

1,4-Dioxane (0.75 L) and 6-chloro-4-((2-methoxy-3-(1-methyl-1*H*-1,2,4-triazol-3-yl)phenyl)amino)-*N*-(trideuteromethyl)pyridazine-3-carboxamide (Xa; 50.00 g, 132.69 mmol) were charged under argon atmosphere into a 3 L glass-jacketed reactor at 25-30 °C. The resulting solution was heated to 45-50 °C and stirred under such conditions for 20 min, followed by degassing with argon for 30 min and then, cooling down to 30-35 °C. Cyclopropanecarboxamide (16.93 g, 199.03 mmol) and solid potassium phosphate (70.41 g, 331.72 mmol) were loaded to the reactor and the resulting mixture was degassed with argon for 30 min. Subsequently, tris(dibenzylideneacetone)dipalladium(0) ((Pd₂(dba)₃), 9.11 g, 9.952 mmol) and 1,1'-ferrocenediyl-bis(diphenylphosphine) (dppf, 11.12 g, 19.90 mmol) were added over the previous mixture which was further degassed for 20 min. The reaction mixture was heated to 95-100 °C (reflux) and further stirred at this temperature for 24 h. Afterwards, the mixture was cooled down to 25-30 °C and diluted with 1:5 EtOH/DCM (1.5 L). After stirring for 10 min at 25-30 °C, the mixture was filtered through a thin pad of Celite and the filter cake was washed gently with 1:5 EtOH/DCM (3 x 0.50 L). The filtrate was first concentrated under reduced pressure (final volume approx. 0.10 L), followed by solvent swap twice with EtOAc (2 x 0.25 L) and subsequent distillations (each final volume approx. 0.10 L). The thus obtained slurry was further diluted with EtOAc (0.50 L), followed by treatment with aqueous HCI solution (0.75 L, 530.76 mmol) [HCI solution: 52 mL HCl in 698 mL of water]. After stirring at 25-30 °C for 30 min, the first aqueous layer was separated while the organic layer was further diluted with aqueous HCI solution (0.38 L, 265.38 mmol) [HCI solution: 26 mL HCI in 349 mL of water]. After stirring at 25-30 °C for 20 min, the second aqueous layer was separated. The combined aqueous layers were then filtered, and the aqueous filtrate was washed twice with EtOAc (0.50 L + 0.25 L). The resulting aqueous solution was diluted with 1:5 EtOH/DCM (1.5 L) followed by adjustment to pH 7.0-7.5 by addition of aqueous K₂HPO₄ solution (600 g in 1.2 L of water) at 25-30 °C. After that, phases were separated, and the aqueous layer was back extracted with 1:5 EtOH/DCM (0.25 L). The combined organic layers were microfiltered. The resulting clear solution was first concentrated under reduced pressure (final volume approx. 0.10 L), followed by solvent swap twice with EtOH (2 x 0.25 L) and subsequent distillations (each final volume approx. 0.10 L). The thus obtained slurry was further diluted with EtOH (1.0 L), followed by heating to 70-75 °C and stirring at this temperature for 0.5 h. At this point, water (2.5 L) was added at 70-75 °C and stirring was continued at the same temperature for additional 1 h period. The resulting hazy solution was microfiltered at 70-75 °C and transferred to a 5 L glass-jacketed reactor. After 0.5 h at 70-75 °C, the stirred solution was seeded with Deucravacitinib Form A (0.50 g) and further stirred at 70-75 °C for additional 20 min, followed by cooling down to 25-30 °C for 1.5 h and further stirring at 25-30 °C for 1 h. The resulting slurry was then cooled to 0-5 °C, stirred for 0.5 h at 0-5 °C and filtered. The wet cake was first washed with 44:56 EtOH/water (50 mL) and then, washed with EtOH (50 mL). After drying under vacuum at 50 °C for 10 h, it was isolated crude deucravacitinib as a yellow solid (33.0 g, 58% yield; HPLC purity: 98% a/a; HPLC RT: 8.3 min).

### Recrystallization of crude deucravacitinib (purification & polymorphic form setting step):

Crude deucravacitinib (33.0 g, 77.56 mmol) was further suspended in ethanol (0.66 L). The suspension was heated to 75-80 °C and further stirred at this temperature for 0.5 h. At this point, water (1.65 L) was added slowly at 75-80 °C, followed by stirring under the same conditions for additional 1 h. The resulting solution was cooled to 70-75 °C. After 0.5 h at 70-75 °C, the solution was seeded with Deucravacitinib Form A (0.33 g) and further stirred at 70-75 °C for additional 20 min, followed by cooling down to 25-30 °C for 1 h and further stirring at 25-30 °C for 1 h. The resulting slurry was then cooled to 0-5 °C, stirred for 0.5 h at 0-5 °C and filtered. The wet cake was first washed with 44:56 EtOH/water (33 mL) and then, washed with EtOH (33 mL). After drying under vacuum at 50 °C for 6 h, it was isolated Deucravacitinib Form A as a light yellow solid (25.4 g, 77% yield; HPLC purity: >99% a/a, HPLC RT: 8.3 min). ¹H NMR (400 MHz, DMSO-*d*₆) *δ* 0.75-0.90 (m, 4H, 2C*H*₂, cyclopropyl), 2.04-2.11 (m, 1H, C*H*, cyclopropyl), 3.72 (s, 3H, NC*H*₃), 3.95 (s, 3H, OC*H*₃), 7.27 (dd, *J* = 7.6 and 7.6, 1H, *H*_{Ar}, phenyl), 7.51 (dd, *J* = 7.6 and 1.6, 1H, *H*_{Ar}, phenyl), 7.65 (dd, *J* = 7.6 and 1.6, 1H, *H*_{Ar}, phenyl), 8.15 (s, 1H, *H*_{Ar}, pyridazinyl), 8.56 (s, 1H, *H*_{Ar}, triazolyl), 9.13 (bs, 1H, CD₃N*H*CO), 11.0 (bs, 1H, N*H*, amine/amide), 11.3 (bs, 1H, N*H*, amine/amide); MS (ESI+): *m*/*z* 426.36 [M + H]⁺. ¹H NMR (400 MHz, DMSO-*d*₆) *δ* 0.75-0.90 (m, 4H, 2C*H*₂, cyclopropyl), 2.04-2.11 (m, 1H, C*H*, cyclopropyl), 3.72 (s, 3H, NC*H*₃), 3.95 (s, 3H, OC*H*₃), 7.27 (dd, *J* = 7.6 and 7.6, 1H, *H*_{Ar}, phenyl), 7.51 (dd, *J* = 7.6 and 1.6, 1H, *H*_{Ar}, phenyl), 7.65 (dd, *J* = 7.6 and 1.6, 1H, *H*_{Ar}, phenyl), 8.15 (s, 1H, *H*_{Ar}, pyridazinyl), 8.56 (s, 1H, *H*_{Ar}, triazolyl), 9.13 (bs, 1H, CD₃N*H*CO), 11.0 (bs, 1H, N*H*, amine/amide), 11.3 (bs, 1H, N*H*, amine/amide); MS (ESI+): *m*/*z* 426.36 [M + H]⁺.

### Comparative example: Preparation of 6-chloro-4-((2-methoxy-3-(1-methyl-1H-1,2,4-triazol-3-yl)phenyl)amino)-N-(trideuteromethyl)pyridazine-3-carboxamide

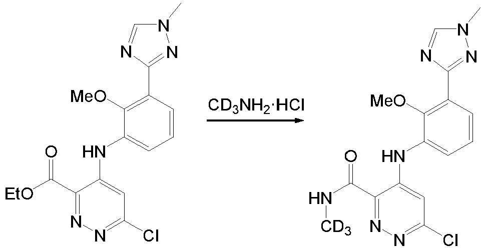

Tetrahydrofuran (5.0 mL) and ethyl 6-chloro-4-((2-methoxy-3-(1-methyl-1*H*-1,2,4-triazol-3-yl)phenyl)amino)pyridazine-3-carboxylate (0.5 g, 1.29 mmol) were charged under nitrogen atmosphere into a RBF at 25-30 °C. Trideuteromethylamine hydrochloride (109 mg, 1.54 mmol) was added at 25-30 °C over the previously prepared stirred suspension under nitrogen atmosphere. The resulting suspension was cooled to 20 °C, and then, lithium bis(trimethylsilyl)amide, 1.3 M solution in tetrahydrofuran (1.48 mL, 1.93 mmol) was added dropwise. The reaction mixture was stirred at 20 °C for 1 h. Afterwards, the mixture was quenched by slow addition of 2:1 (v/v) water/saturated aqueous NH₄Cl solution (7.5 mL), followed by phase separation. The separated organic layer was concentrated to dryness under reduced pressure to render 6-chloro-4-((2-methoxy-3-(1-methyl-1*H*-1,2,4-triazol-3-yl)phenyl)amino)-N-(trideuteromethyl)pyridazine-3-carboxamide (Xa) as a light brown solid (160 mg, 33.0% yield; HPLC purity: 89% a/a; HPLC RT: 9.3 min). Note: HPLC analysis of the separated aqueous layer confirmed the presence of free carboxylic acid derivative formed through undesired ester hydrolysis as a major reaction side product (HPLC RT: 8.3 min; 40% content found in the crude mixture), which was also the main impurity present in the amidation product (8% content in the isolated solid).

### Citation List

WO2014/0074661
WO2018/0183649
WO2018/0183656
WO2023/102085

## Claims

1. A process for the preparation of deucravacitinib of formula (XI) or a pharmaceutically acceptable salt thereof, 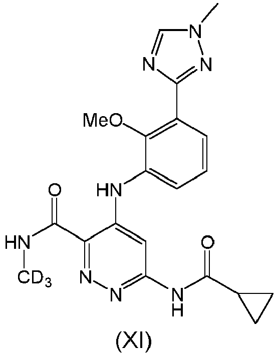 which comprises:
a) reacting a compound of formula (IX), or a salt thereof, 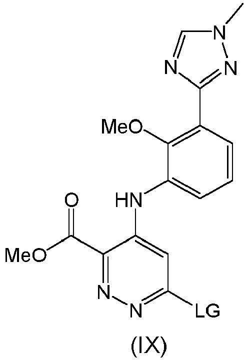 with methyl-*d*₃-amine, or a salt thereof, in the presence of cesium carbonate in dimethylformamide to give a compound of formula (X) or a salt thereof; 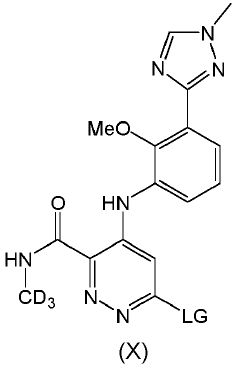 wherein LG is a leaving group selected from the group consisting of halogen, -OSO₂R₁, and -OCOR₁, and R₁ is selected from the group consisting of -(C₁-C₄)alkyl, -(C₁-C₄)haloalkyl, -(C₁-C₄)alkyl(C₆-C₁₂)aryl, -(C₁-C₄)alkyl(C₄-C₁₂)heteroaryl, -(C₆-C₁₂)aryl, and -(C₄-C₁₂)heteroaryl; wherein the (C₆-C₁₂)aryl and (C₄-C₁₂)heteroaryl moieties are optionally substituted with one or more (C₁-C₄)alkyl or (C₁-C₄)haloalkyl groups;
b) reacting the compound of formula (X), or a salt thereof, with cyclopropanecarboxamide, or a salt thereof, to give a compound of formula (XI); and
c) optionally converting deucravacitinib of formula (XI) into a pharmaceutically acceptable salt thereof.

2. The process according to claim 1, wherein in the compound of formula (IX), LG is chloro, *i.e.,* the compound of formula (IX) is a compound of formula (IXa):

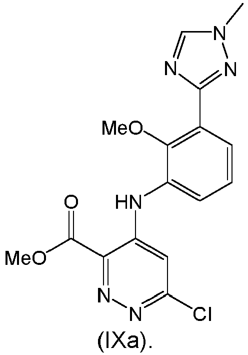

3. The process according to any of claims 1 to 2, wherein in step a) methyl-*d*₃-amine and cesium carbonate are used each in an amount from 1 to 5 molar equivalents, relative to the compound of formula (IX).

4. The process according to any of claims 1 to 3, wherein previously to step a), the process comprises:
vii) reacting the compound of formula (VIII), or a salt thereof, 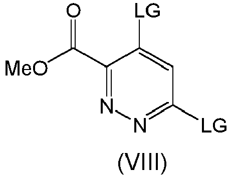 wherein each LG is independently as defined in claim 1; with a compound of formula (VI), or a salt thereof, 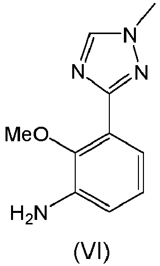 to obtain a compound of formula (IX) or a salt thereof.

5. The process according to claim 4, wherein previously to step vii), the process comprises:
vi) converting a compound of formula (VII), or a salt thereof, 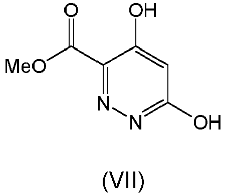 into compound of formula (VIII), or a salt thereof; 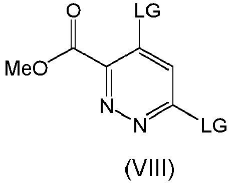 wherein each LG is independently as defined in claim 1.

6. The process according to claim 5, wherein in the compound of formula (VIII), each LG is chloro, *i.e.,* the compound of formula (VIII) is a compound of formula (Villa):

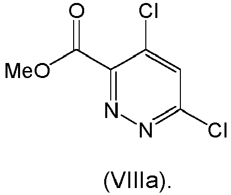

7. The process according to any of claims 4 to 6, wherein previously to step vii), the process comprises:
v) reacting a compound of formula (V'), or a salt thereof, 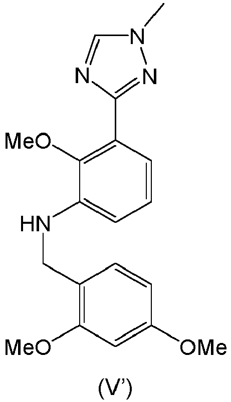 with an acid to give a compound of formula (VI), or a salt thereof; or alternatively previously to step vii), the process comprises:
v') reacting a compound of formula (V"), or a salt thereof, 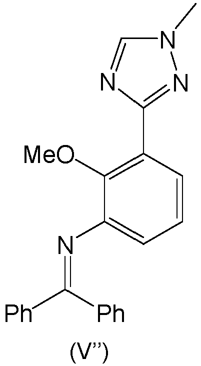 with an acid to give a compound of formula (VI), or a salt thereof.

8. The process according to claim 7, wherein previously to step v) the process comprises:
iv) reacting the compound of formula (IV), or a salt thereof, 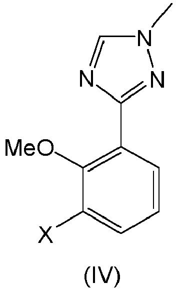 wherein X is halogen with 2,4-dimethoxybenzylamine or a salt thereof, in presence of metallic catalyst to give a compound of formula (V'), or a salt thereof;
or alternatively previously to step v'), the process comprises:
iv') reacting the compound of formula (IV), or a salt thereof, wherein X is halogen, with benzophenone imine, in presence of metallic catalyst to give a compound of formula (V"), or a salt thereof.

9. The process according to claim 8, wherein previously to step iv) or alternatively step iv') the process comprises:
iii) reacting the compound of formula (III), or a salt thereof, 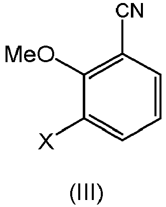 wherein X is halogen, with *N*-formyl-*N*-methylhydrazine, or a salt thereof, to give a compound of formula (IV), or a salt thereof.

10. The process according to claim 9, wherein previously to step iii) the process comprises:
ii) converting a compound of formula (II), or a salt thereof, 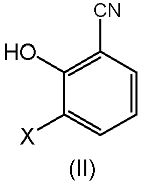 wherein X is halogen; into the compound of formula (III), or a salt thereof.

11. The process according to claim 10, wherein previously to step ii) the process comprises:
i) halogenating a compound of formula (I), or a salt thereof, 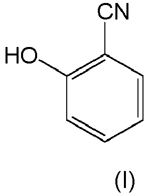 to give a compound of formula (II), or a salt thereof.

12. A process for the preparation of a compound of formula (V'), or a salt thereof 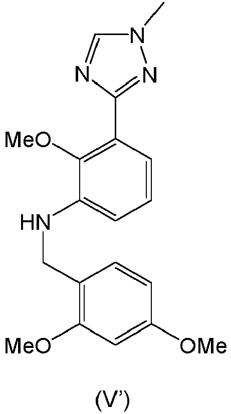 which comprises
iv) reacting the compound of formula (IV), or a salt thereof, with 2,4-dimethoxybenzylamine or a salt thereof, in presence of metallic catalyst.

13. A process for the preparation of a compound of formula (V"), or a salt thereof 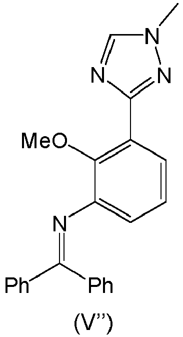 which comprises:
iv') reacting the compound of formula (IV), or a salt thereof, with benzophenone imine, in presence of metallic catalyst.

14. A compound of formula (V'), or a salt thereof:

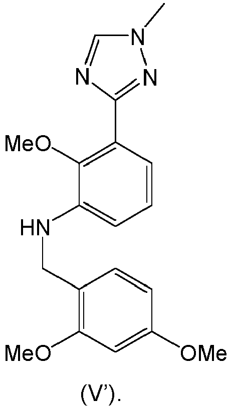

15. A compound of formula (V"), or a salt thereof:
